# EUROPÄISCHE PATENTSCHRIFT

(11) **EP 1 539 925 B1**
(45) Veröffentlichungstag und Bekanntmachung des Hinweises auf die Patenterteilung: **17.10.2007**
(21) Anmeldenummer: 02807963.0
(22) Anmeldetag: 16.09.2002
(51) Int. Cl.: C12M 3/06, C12N 5/00

(54) **ZELLKULTURKAMMER FÜR EIN ZELLKULTURSYSTEM**
CELL CULTURE CHAMBER FOR A CELL CULTURE SYSTEM
CHAMBRE DE CULTURE CELLULAIRE POUR SYSTEME DE CULTURE CELLULAIRE

(43) Veröffentlichungstag der Anmeldung: 15.06.2005
(73) Patentinhaber: Pan - Biotech GmbH, 94501 Aidenbach (DE)
(72) Erfinder: SEIDL, Josef, 94474 Vilshofen a.d. Donau (DE); SCHERZE, Wilhelm, 90765 Fürth (DE)
(74) Vertreter: Benninger, Johannes
(86) Internationale Anmeldenummer: PCT/EP2002/010359
(87) Internationale Veröffentlichungsnummer: WO 2004/033617

(56) Entgegenhaltungen:
- WO-A-00/78920
- WO-A-96/00780
- US-A- 5 707 869
- US-A- 5 958 762

## Beschreibung

Die Erfindung betrifft eine Zellkulturkammer für ein geschlossenes Zellkultursystem zur kontinuierlichen Versorgung verschiedenster Zellen mit flüssigen Nährmedien, Wachstumsfaktoren, Gasen und dergleichen.

Unter Zellkultur versteht man im wesentlichen eine Kultur, die von einzelnen Zellen angesetzt wird, die entweder von Gewebeteilen, von primären Kulturen, von Zell-Linien oder Zell-Stämmen durch enzymatische, mechanische oder chemische Zerteilung herrühren. Zur Kultivierung der Zellen werden üblicherweise Kulturgefäße aus Plastik verwendet, die in CO₂-Brutschränken inkubiert werden. Diese garantieren eine konstante Temperatur (z.B. 37°C) und eine Pufferung des Mediums durch eine 5 %- bis 10 %-ige Ca₂-Begasung. Die Sauerstoffversorgung erfolgt durch einfache Diffusion. Bei den bekannten Einrichtungen sind Co-Kultivierung und frei veränderliche Inkubationsbedingungen in der Regel nicht möglich.

Zur mikroskopischen Beobachtung oder zu speziellen Untersuchungen müssen die Kulturgefäße aus dem jeweiligen Brutschrank entnommen werden, wobei die Inkubation unterbrochen wird, die Zellen sich abkühlen und somit die Versuchsbedingungen nicht mehr konstant sind.

Die bisher bekannten Zellkultureinrichtungen oder Zellkultursysteme werden jedoch den Anforderungen der modernen Zellkulturtechnologie nicht mehr gerecht.

Insbesondere im Hinblick auf aktuelle Forschungsschwerpunkte in der Pharmaindustrie, die in den Bereichen Entzündung (Rheuma), Krebsbekämpfung, Herz/Kreislauf-Erkrankungen, Aids, Apoptose (programmierter Zelltod) und Blutgerinnung liegen, ist die Entwicklung und Erprobung entsprechender neuer Wirkstoffe und Medikamente mit Hilfe eines Zellkultursystems unabdingbar, das in der Weise konzipiert sein soll, daß die Substanz- und Wirkungstestung unter nahezu in-vivo-Bedingungen, d.h., mit nahezu perfekter Abbildung komplexer biologischer Systeme, vor Übertritt in die klinischen Phasen (Testung an Probanten) ermöglicht wird.

Mit Rücksicht auf die geschilderte Situation besteht die Forderung nach einer Möglichkeit der Simulation von Reaktionsabläufen innerhalb eines oder mehrerer Organsysteme (z.B. durch Serienschaltung von Zellkulturkammern mit Hepatozyten und anderen Zellarten, Untersuchung auf Abbauprodukte und Metabolite), damit zum einen die Zeiträume zwischen Substanzwirkungserkennung und Arzneimittelzulassung erheblich minimiert werden und zum anderen vor dem Eintritt in die klinische Testphase die notwendigen Erkenntnisse über den Wirkungsmechanismus der Substanz innerhalb eines komplexen biologischen Systems erlangt werden können.

Eine ähnliche Situation liegt beispielsweise auch im Bereich der Kosmetikindustrie vor.

Im Stand der Technik sind beispielsweise multivalente Zellkultursysteme (vgl. z.B. DE 199 15 178 A1), problemadaptierte Zellkultursysteme für spezifische Aufgabenstellungen (vgl. z.B. WO 98/17822) oder Verfahren zur Replikation von Zellkulturen bekannt (vgl. z.B. WO 97/37001).

Ferner ist beispielsweise aus der WO 99/23206 ein Verfahren zum Mischen einer varizella-infizierten Zellkultur in Rollflaschen bekannt.

Schließlich ist aus der EP 0 999 266 A1 ein Verfahren und eine Vorrichtung zur Aufnahme einer Zellkultur bekannt, wodurch möglichst homogene Bedingungen für die molekularbiologische oder gentechnische Untersuchung von Zellen geschaffen werden sollen.

Mit Rücksicht auf die eingangs geschilderte Situation auf dem Gebiet der modernen Zellkulturtechnologie liegt der vorliegenden Erfindung nunmehr die Aufgabe zugrunde, eine neue, zur Aufnahme wenigstens einer Zellkultur dienende Zellkulturkammer für ein geschlossenes Zellkultursystem zu schaffen, wobei eine kontinuierliche Versorgung insbesondere verschiedener Zellen mit flüssigem. Nährmedium, Wachstumsfaktoren, Gasen und dergleichen in der Zellkulturkammer gewährleistet ist, ohne daß die Zellen ihrer gewohnten Umgebung entnommen werden müssen, und wobei ferner eine permanente mikroskopische Beobachtung der Zellkulturen ohne Unterbrechung der Begasung ermöglicht ist.

Diese Aufgabe wird bei einer Zellkulturkammer für ein geschlossenes Zellkultursystem zur kontinuierlichen Versorgung unterschiedlicher Zellen mit flüssigen Nährmedien, Wachstumsfaktoren, Gasen und dergleichen erfindungsgemäß dadurch gelöst, daß die Zellkulturkammer im wesentlichen aus den folgenden Komponenten aufgebaut ist:
a) eine Membranplatte mit einer Membran zur Aufnahme wenigstens einer Zellkultur und mit einer Anzahl von Kanälen für Flüssigkeitszufuhr, Begasung und Sensorikanschluß;
b) eine auf der einen Seite der Membranplatte angeordnete lichtdurchlässige Glasscheibe für eine Beobachtung des Inneren der Zellkulturkammer von der genannten einen Seite aus; und
c) eine auf der anderen, gegenüberliegenden Seite der Membranplatte angeordnete Abschlußplatte mit eingebauter, lichtdurchlässiger Glasscheibe für eine Beleuchtung des Inneren der Zellkulturkammer von der genannten anderen Seite aus mit Hilfe eines zugeordneten Beleuchtungssystems.

Vorzugsweise ist hierbei die lichtdurchlässige Glasscheibe an der Membranplatte für die Beobachtung des Inneren der Zellkulturkammer im Bereich der Unterseite der Membranplatte befestigt.

Ferner bildet in bevorzugter Weise die Abschlußplatte einen Zellkulturkammerdeckel mit einer fest integrierten, lichtdurchlässigen Glasscheibe, wobei der Zellkulturkammerdeckel an der Oberseite der Membranplatte lösbar angebracht ist.

Gemäß weiterer Ausgestaltung der Erfindung ist vorgesehen, daß sowohl der Zellkulturkammerdeckel als auch die Unterseite der Membranplatte eine Ausnehmung zur Aufnahme und Befestigung der entsprechenden Glasscheibe aufweisen, insbesondere zur nichtlösbaren Befestigung.

Vorzugsweise ist die jeweilige lichtdurchlässige Glasscheibe durch eine Saphirglasscheibe gebildet.

Darüberhinaus kann gemäß weiterer Ausgestaltung der Erfindung noch vorgesehen sein, daß zur Fixierung der Membran an der Membranplatte ein Haltering vorhanden ist, der mit Hilfe des Zellkulturkammerdeckels auf den Randbereich der Membran preßbar ist, wodurch die letztere fixierbar ist.

Eine weitere Ausgestaltung der Erfindung besteht darin, daß vorzugsweise auf der der Membranplatte zugewendeten Seite das Zellkulturkammerdeckels ein Dichtring vorgesehen ist, durch den im geschlossenen Zustand der Zellkulturkammer die auf der Membran ausgesäte Zellkultur aspetisch verschlossen wird.

Eine andere, bevorzugte Weiterbildung der Erfindung besteht darin, daß durch eine geeignete Kompartimentierung der Zellkulturkammer eine konstante, kontinuierliche Begasung durch die entsprechend zugeordneten Kanäle hindurch mit frei wählbaren Konzentrationen unterschiedlichster Gase ermöglicht ist. Dies hat insbesondere den Vorteil, daß eine Beobachtung der Zellkultur im Inneren der Zellkulturkammer ohne Unterbrechung der Begasung erfolgen kann.

Darüberhinaus besteht auch die Möglichkeit, daß die Membranplatte auf ihrer dem Zellkulturkammerdeckel gegenüberliegenden Seite an einer zugeordneten Halteplatte zum Einbringen in das Zellkultursystem anbringbar ist, wobei diese Halteplatte eine integrierte Heizung für die Zellkulturkammer aufweist. Vorzugsweise ist diese Heizung eine elektrische Heizung.

Falls eine direkte Co-Kultivierung durchgeführt werden soll, kann mit besonderem Vorteil eine gasdurchlässige Biofolie als Membran verwendet werden, wie weiter unten noch näher erläutert wird.

Die Erfindung wird nunmehr nachfolgend anhand von Ausführungsbeispielen näher erläutert, wobei zeigen:
Figur 1 schematisch eine Draufsicht auf eine Zellkulturkammer;
Figur 2 eine Schnittansicht der Zellkulturkammer gemäß der Linie A-A nach Figur 1 ;
Figur 3 die Schnittansicht der Zellkulturkammer gemäß Figur 2 in auseinandergezogener Darstellung;
Figur 3A schematisch eine Seitenansicht einer Membranplatte der Zellkulturkammer; und
Figur 4 eine schematische Ansicht eines kompletten, geschlossenen Zellkultursystems, bei dem eine vorgegebene Anzahl von Zellkulturkammern zum Einsatz gelangt.

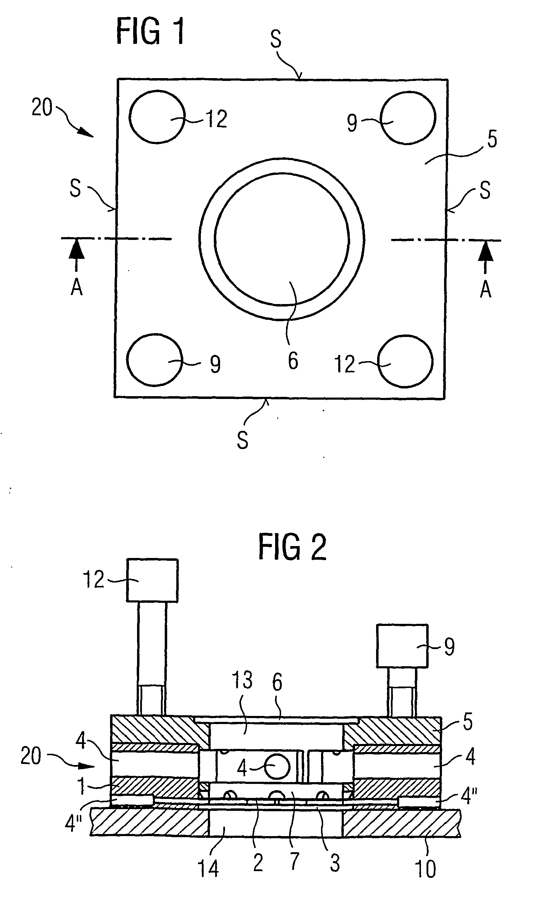

Gemäß Figuren 1 2, 3 und 3A besteht eine Zellkulturkammer 20 im wesentlichen aus einer Membranplatte 1, in die eine Membran 2, insbesondere eine gasdurchlässige Biofolie eingebracht, ist, die zur Aufnahme wenigstens einer Zellkultur dient. Im gezeigten Ausführungsbeispiel ist die Membran 2 im unteren Bereich der Membranplatte 1 angeordnet, insbesondere fest eingespannt.

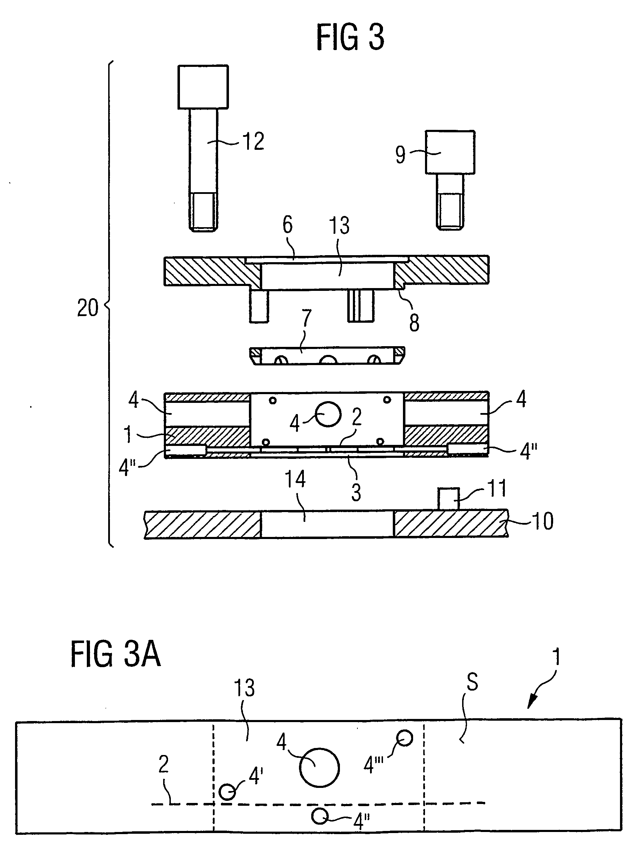

Die Membranplatte 1 weist ferner eine Anzahl von Kanälen 4, 4', 4" und 4"' auf, die im Inneren der Zellkulturkammer 20 verlaufen und von denen der Kanal 4 zum Sensorikanschluß, der Kanal 4' zur Flüssigkeits- oder Gaszufuhr, der Kanal 4" zur Flüssigkeitszufuhr und der Kanal 4 "' zur Flüssigkeits- oder Gas-Ableitung dienen, wie dies weiter unten anhand der Figur 3A noch näher erläutert wird. Die Zellkultur kann somit sowohl von oben als auch von unten gleichermaßen versorgt werden. Durch das spezielle System von Kanälen in der Membranplatte 1 ist insbesondere gewährleistet, daß die erforderlichen Inkubationsbedingungen realisiert werden können.

Im Bereich der Unterseite der Membranplatte 1 ist eine lichtdurchlässige Glasscheibe 3 für eine Beobachtung des Inneren der Zellkulturkammer 20 angeordnet. Eine derartige Beobachtung erfolgt vorzugsweise von der Unterseite der Membranplatte 1 aus mit Hilfe einer Videokamera mit Mikroskopaufsatz, wie dies noch weiter unten erläutert wird.

An der Oberseite der Membranplatte 1 ist ein Zellkulturkammerdeckel 5 angeordnet, der eine obere Abschlußplatte bildet und in den eine lichtdurchlässige Glasscheibe 6 für eine Beleuchtung des Inneren der Zellkulturkammer 20 eingebaut ist. Der Zellkulturkammerdeckel 5 ist insbesondere an der Oberseite der Membranplatte 1 fest angebracht und vorzugsweise mit Hilfe von Schrauben 9 mit der Membranplatte 1 in lösbarer Weise verschraubt.

Sowohl der Zellkulturkammerdeckel 5 als auch die Unterseite der Membranplatte 1 sind mit einer entsprechenden Ausnehmung versehen, um die entsprechende Glasscheibe 6 bzw. 3 aufnehmen und befestigen zu können.

Bei dem Zellkulturkammerdeckel 5 deckt somit die Glasscheibe 6 eine vorzugsweise runde Öffnung 13 ab.
In entsprechender Weise bildet bei der Membranplatte 1 die Glasscheibe 3 einen unteren Abschluß unterhalb der Membran 2.

In bevorzugter Weise sind die lichtdurchlässigen Glasscheiben 3 und 6 jeweils Saphirglasscheiben.

Zur Fixierung der Membran 2 an der Membranplatte 1 ist ein Haltering 7 vorgesehen, der mit Hilfe des Zellkulturkammerdeckels 5 auf den Randbereich der Membran 2 gepreßt werden kann, um somit die letztere in der Zellkulturkammer 20 zu fixieren.

Darüberhinaus ist auf der der Membranplatte 1 zugewendeten Seite des Zellkulturkammerdeckels 5 ein Dichtring 8 angeordnet. Mit Hilfe dieses Dichtringes 8 wird im geschlossenen Zustand der Zellkulturkammer 20 (vgl. Figur 2) die auf der Membran 2 ausgesäte Zellkultur aseptisch verschlossen.

Figur 3A zeigt schematisch eine Seitenansicht der Membranplatte 1 mit dort vorgesehenen Mündungen des Kanals 4 für Sensorikanschluß, des Kanals 4' für Flüssigkeits- oder Gaszufuhr, des Kanals 4" für Flüssigkeitszufuhr und des Kanals 4"' für Flüssigkeits- oder Gas-Ableitung.

Die Mündungen der Kanäle 4, 4', 4" und 4"', wie sie in Figur 3A für eine Seite S der Membranplatte 1 gezeigt sind, sind auf allen drei weiteren Seiten der Membranplatte 1 in identischer Weise vorgesehen.

Aus Figur 3A ist auch die Plazierung der gasdurchlässigen Membran 2 im Inneren der Membranplatte 1 zu ersehen, wobei die Plazierung der Membran 2 so gewählt ist, daß sich eine definierte Kompartimentierung der Zellkulturkammer 20 ergibt, wodurch sich eine direkte Co-Kultivierung zweier Zellkulturen ermöglichen läßt. Bei einer derartigen direkten Co-Kultivierung wird zu beiden Seiten der Membran 2 je eine Zellkultur unterschiedlicher Art angesiedelt, wobei insbesondere die auf der einen Seite der Membran 2, d.h. auf der apikalen Seite wachsenden Zellen der ersten Zellkultur durch einen ersten Medienfluß in den Kanal 4' versorgt werden, wohingegen die auf der anderen Seite der Membran 2, d.h. der basolateralen Seite wachsenden Zellen der zweiten Zellkultur mit einem gegenüber dem ersten Medienfluß unterschiedlichen, zweiten Medienfluß durch den Kanal 4" versorgt werden. Somit funktionieren die Zellen auf der apikalen Seite als Deckschicht, während die Zellen auf der basolateralen Seite als Innenzellen funktionieren.

Der zur apikalen Seite führende Kanal 4' kann aber auch zur Begasung dienen, insbesondere zu einer konstanten, kontinuierlichen Begasung mit frei wählbaren Konzentrationen unterschiedlichster Gase.

Der Kanal 4 dient, wie bereits erwähnt, für den Sensorikanschluß.

Der Kanal 4 "' dient schließlich für die Ableitung von Flüssigkeiten oder Gasen von der apikalen Seite der Membran 2.

Die Komponenten der Zellkulturkammer 20 sind insbesondere aus einem geeigneten Edelstahl gefertigt, beispielsweise aus Edelstahl 1,4435.

Nach Bestückung der Membranplatte 1 im Reinraum wird der Zellkulturkammerdeckel 5 aufgebracht und mit Hilfe der Schrauben 9 mit der Membranplatte 1 verschraubt, wobei es sich hierbei um kurze Schrauben 9 handelt, welche den Zellkulturkammerdeckel 5 an der Membranplatte 1 fixieren. Hierbei wird gleichzeitig mit Hilfe des Dichtrings 8 die Zellkultur, die von der Membranplatte 1 beherbergt wird, aspetisch verschlossen.

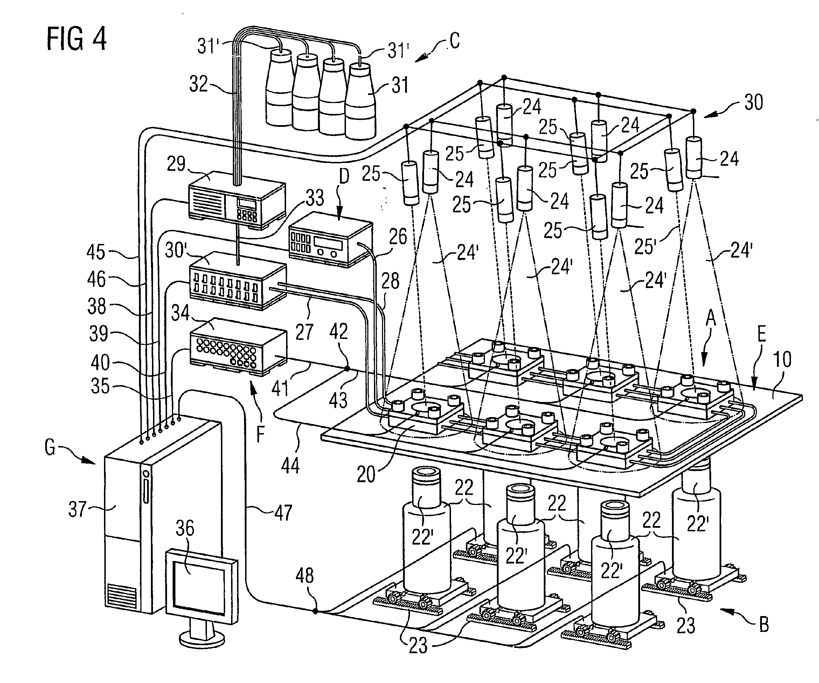

In diesem Zustand wird die Zellkulturkammer 20 mit einer Halteplatte 10 eines Zellkultursystems (vgl. Fig. 4) zusammengebaut.

Insbesondere wird zu diesem Zweck die Membranplatte 1 an ihrer zu dem Zellkulturkammerdeckel 5 entgegengesetzten Seite an der Halteplatte 10 angebracht, die einen Aufnahmebolzen 11 für eine Justierung aufweist. Zur Befestigung der Membranplatte 1 an der Halteplatte 10 sind relativ lange Schrauben 12 vorgesehen. Die Halteplatte 10 weist ferner eine integrierte Heizung, vorzugsweise eine elektrische Heizung, für die Zellkulturkammer 20 auf, wie weiter unten noch näher erläutert wird.

Im gezeigten Ausführungsbeispiel ist die Zellkulturkammer 20 im wesentlichen quaderförmig ausgebildet und weist einen quadratischen Grundriß auf. Selbstverständlich sind auch noch andere geometrische Ausführungen denkbar.

Im anhand der Fig. 1 - 3A erläuterten Ausführungsbeispiel sind Mündungen der Kanäle 4, 4', 4" und 4"' auf allen vier Seiten S der Membranplatte 1 in gleicher Weise vorgesehen. Aber auch hier wären noch andere Anordnungen für diese Kanäle, die im wesentlichen zylindrisch sind, denkbar. Andere Kanalquerschnitte sind ebenfalls denkbar.

Es ist noch zu erwähnen, daß die Halteplatte 10 für jede an dieser anzubringende Zellkulturkammer 20 eine mittlere, kreisrunde Öffnung 14 aufweist, deren Durchmesser dem Öffnungsbereich 13 des gegenüberliegenden Zellkulturkammerdeckels 5 entspricht. Diese mittlere Öffnung 14 der Halteplatte 10 gewährleistet die Beobachtung des Inneren der Zellkulturkammer 20 von unten mit Hilfe einer Videokamera mit Mikroskopaufsatz, wie dies anhand der Figur 4 noch erläutert wird.

Figur 4 veranschaulicht die Anwendung der erfindungsgemäßen Zellkulturkammern 20 bei einem geschlossenen Zellkultursystem 30.

Bei diesem Zellkultursystem 30 sind beispielsweise sechs Zellkulturkammern 20 als Gruppe A auf der Halteplatte 10 plaziert, die durch ihre integrierte Heizung E für die Inkubierung während der Betriebszeit des Zellkultursystems 30 konstante Temperaturen innerhalb jeder der Zellkulturkammern 20 der Zellkulturkammergruppierung A gewährleistet.

Insbesondere erfolgt mit Hilfe dieser Heizung E eine elektrische Beheizung der jeweiligen Zellkulturkammer 20, wodurch eine sehr genaue Temperaturregelung ermöglicht ist. Die Heizung E ist insbesondere in der Weise ausgelegt, daß jede einzelne Zellkulturkammer 20 der Zellkulturkammergruppierung A gleichsam individuell beheizt werden kann.

Ein besonderer Vorteil des Zellkultursystems 30 liegt darin, daß die Heizung E über eine zugeordnete Software steuerbar ist. Zu diesem Zweck ist oberhalb der Zellkulturkammergruppierung A ein System aus Infrarot-Temperaturmessern 25 installiert, in der Art, daß jeder einzelnen Zellkulturkammer 20 ein entsprechender Infrarot-Temperaturmesser 25 zugeordnet ist. Der jeweilige Infrarot-Temperaturmesser 25 fühlt mit Hilfe eines von der jeweiligen Zellkulturkammer 20 ausgehenden Infrarotstrahls 25' die in der Zellkultur vorherrschende Temperatur ab und meldet das entsprechende Meßergebnis permanent an ein computergesteuertes Überwachungs- und Steuerungssystem G, das im wesentlichen aus einer Datenverarbeitungsanlage 37 und einem Monitor 36 besteht. Die einzelnen Infrarot-Temperaturmesser 25 sind über eine gemeinsame Verbindungsleitung 45 an das Überwachungs- und Steuerungssystem G angeschlossen. Wenn sich die anfangs vorgegebenen Temperaturen in den Zellkulturkammern 20 der Zellkulturkammergruppierung A ändern, erfolgt automatisch über das Überwachungs- und Steuerungssystem G eine Steuerung bzw. Regelung der Heizung E, d.h., die in der einzelnen Zellkulturkammer 20 herrschende Temperatur wird permanent auf eine konstante Temperatur eingeregelt. Anstatt mit Hilfe von Infrarot-Temperaturmessern könnte die Temperaturmessung auch mittels anderer geeigneter Temperatur-Sensoren erfolgen.

Andererseits kann mit Hilfe der in dem Überwachungs- und Steuerungssystem G enthaltenen Software ermöglicht werden, daß die Temperaturen in den einzelnen Zellkulturkammern 20 der Zellkulturkammergruppierung A während der gesamten Versuchsdauer frei einstellbar und veränderbar sind, falls dies aus bestimmten Gründen erforderlich sein sollte.

Zum Zwecke der permanenten mikroskopischen Beobachtung des Inneren der jeweiligen Zellkulturkammer 20 ist ein Videosystem B mit einem entsprechend zugeordneten Mikroskopsystem vorgesehen. Dieses Videosystem B wird im folgenden näher erläutert.

Unterhalb jeder einzelnen Zellkulturkammer 20 der Zellkulturkammergruppierung A, die im vorliegenden Ausführungsbeispiel insgesamt sechs Zellkulturkammern aufweist, ist eine Videokamera 22 mit Mikroskopaufsatz 22' auf einem mechanisch einstellbaren Fahrtisch 23 angeordnet, somit insgesamt sechs Videokameras 22 mit zugehörigem Mikroskopaufsatz 22'. Somit beobachtet je eine Videokamera 22 mit Mikroskopaufsatz 22' je eine Zellkulturkammer 20. Nach Versuchsstart und nachdem sich aussagekräftige Bereiche in der jeweiligen in der Zellkulturkammer 20 enthaltenen Zellkultur abzeichnen, wird ein Beobachtungssektor in der Zellkulturkammer 20 festgelegt. Dieser Beobachtungssektor wird sodann durch den mechanisch einstellbaren Fahrtisch 23 mittels (nicht dargestellter) Einstellschrauben angefahren, sodann wird der Fahrtisch 23 arretiert und das Videosystem B bleibt infolgedessen während der gesamten Versuchsdauer in der gleichen Position. Ferner wird bei Versuchsstart die Schärfe der Einstellung am jeweiligen Mikroskopaufsatz 22' einjustiert. Dieser Justiervorgang am jeweiligen Mikroskopaufsatz 22' erfolgt für sämtliche sechs Zellkulturkammern 20 und bleibt sodann unverändert bis zum Versuchsende.

Vorzugsweise wird auch das Videosystem B über die im Überwachungs- und Steuerungssystem G enthaltene Software gesteuert. Hierbei wird jede einzelne Videokamera 22 mit Mikroskopaufsatz 22' gesteuert. Dies erfolgt insbesondere in der Art, daß in frei wählbaren Zeitintervallen (beispielsweise im Minutentakt) Bilder von der jeweiligen Zellkultur in der Zellkulturkammer 20 aufgenommen werden, wobei zu dem jeweiligen Zeitpunkt einer solchen Aufnahme eine oberhalb der jeweiligen Zellkulturkammer 20 angeordnete Lichtquelle 24 die entsprechende Zellkultur beleuchtet, so daß eine ausreichende Ausleuchtung des Inneren der Zellkulturkammer 20 für die Videoaufnahmen gewährleistet ist. Wenn die Videoaufnahme beendet ist, bringt die Steuerung die jeweilige Lichtquelle 24 in einen schwach dimmenden Standby-Zustand, bis die nächste Videoaufnahme gemacht wird. Der von einer jeden Lichtquelle 24 ausgehende Lichtstrahl bzw. Lichtkegel, der durch die jeweilige Saphirglasscheibe 6 einer Zellkulturkammer 20 in das Innere dieser Zellkulturkammer 20 eintritt, ist in Figur 4 mit 24' bezeichnet.

Sämtliche Lichtquellen 24 sind über eine gemeinsame Verbindungsleitung 46 an das Überwachungs- und Steuerungssystem G angeschlossen.

Durch jeden einzelnen Lichtstrahl bzw. Lichtkegel 24' wird die jeweilige, in der Zellkulturkammer 20 enthaltene Zellkultur flächendeckend ausgeleuchtet.

Das Videosystem B ist ebenfalls über eine Leitung 47 an das Überwachungs- und Steuerungssystem G angeschlossen, wobei von diesem aus die Leitung 47 zu einem Knotenpunkt 48 führt, mit dem die einzelnen Videokameras 22 über entsprechend zugeordnete Leitungen verbunden sind.

Das wie oben erläuterte Videosystem B mit Mikroskopsystem stellt nur eine Ausführungsmöglichkeit dar. Eine mögliche andere Ausführungsform eines solchen Systems zur permanenten Beobachtung des Inneren der Zellkulturkammern besteht darin, daß ein einziges Beobachtungssystem, bestehend aus Videokamera und Mikroskopaufsatz, auf einem Fahrtisch installiert wird und daß dieser Fahrtisch die sechs Zellkulturkammern 20 der Zellkulturkammergruppierung A in frei wählbaren Intervallen abfährt. Die Justierung des Beobachtungssystems erfolgt für die einzelne Zellkultur bei Versuchsstart, d.h., vorzugsweise dann nachdem sich aussagekräftige Bereiche in der jeweiligen Zellkultur abzeichnen, durch die entsprechende, im Überwachungs- und Steuerungssystem G enthaltene Software, d.h., durch das entsprechende Computerprogramm sind die sechs Anfahrpositionen des Fahrtisches, auf dem das Beobachtungssystem montiert ist, programmiert. Wegen der mechanischen Toleranzen des Fahrtisches muß jedoch ein größerer als der zu beobachtende Bereich innerhalb der einzelnen Zellkulturkammer aufgenommen werden. Innerhalb dieses größeren Bereiches wird nun mittels der Software der zu beobachtende Bereich definiert. Die Software ist in der Lage, Konturen zu speichern und wieder zu erkennen, d.h., beim erneuten Anfahren einer Zellkulturkammer wird die Kontur und Anordnung der Zellen erkannt und ein anfänglich definierter Beobachtungsbereich gespeichert.

Dieses zuletzt erläuterte Beobachtungssystem ist in den Zeichnungen im einzelnen nicht dargestellt, jedoch erfolgt die Ausleuchtung des Inneren der Zellkulturkammern 20 ebenfalls mit Hilfe der Lichtquellen 24, wie bereits weiter oben im einzelnen erläutert.

Das in Figur 4 dargestellte Zellkultursystem 30 weist ferner noch ein Dosiersystem C für Flüssigkeiten (z.B. flüssige Nährmedien und dergleichen) auf, welches z.B. vier Flüssigkeitsvorratsbehälter 31 mit einer jeweils zugeordneten Flüssigkeitsentnahmeleitung 31' aufweist, wobei sodann die vier Flüssigkeitsentnahmeleitungen 31' zu einem Leitungsbündel 32 zusammengefaßt sind. Dieses Leitungsbündel 32 ist andererseits mit einem Pumpensystem 29 verbunden, durch welches die verschiedenen Zellkulturkammern 20 der Zellkulturkammergruppierung A mit frei wählbaren Flüssigkeiten, die in den Flüssigkeitsvorratsbehältern 31 enthalten sind, versorgt werden.

Das Pumpensystem 29 ist über eine Leitung 33 an ein Multiventilmodul 30' angeschlossen. Die Zuführung der Flüssigkeiten zu der Zellkulturkammergruppierung A erfolgt von dem Multiventilmodul 30' aus über sterile Schlauchsysteme 27 und 28, wobei diese Flüssigkeiten von den einzelnen Zellkulturkammern 20 flexibel weitergeleitet werden. Sowohl die Flüssigkeitszuführung als auch die Flüssigkeitsab- bzw. weiterleitung erfolgt über sterile Schlauchsysteme, die mit Standard-Schlauchverbinderelementen und Verteilern bei Versuchsstart installiert werden, d.h. mit entsprechenden Kanälen in der Membranplatte 1 einer jeweiligen Zellkulturkammer 20 verbunden werden. Hierbei wird die Verbindung der Standard-Schlauchverbinderelemente (in den Zeichnungen im einzelnen nicht dargestellt) mit den zugeordneten Kanälen der Membranplatte 1 so aufeinander abgestimmt, daß die Sterilität gewährleistet ist.

Aus Gründen der Flexibilität können die Flüssigkeiten, die Strömungsrichtungen, die Verteilung der Flüssigkeiten und deren Durchflußmengen während des Versuchs geändert bzw. gesteuert werden, wobei eine derartige Steuerung vorzugsweise durch das computergesteuerte Überwachungs- und Steuerungssystem G erfolgt. Zu diesem Zweck sind das Pumpensystem 29 mittels einer Verbindungsleitung 38 und das Multiventilmodul 30' über eine Verbindungsleitung 40 an das Überwachungs- und Steuerungssystem G angeschlossen.

Das.Dosiersystem C des Zellkultursystems 30 erlaubt es somit, der Zellkulturkammergruppierung A unterschiedlichste Flüssigkeiten zuzuführen.

Das Zellkultursystem 30 weist darüberhinaus ein Begasungssystem D für unterschiedlichste Gase auf. Dieses Begasungssystem D dient also dazu, die verschiedenen Zellkulturkammern 20 der Zellkulturkammergruppierung A mit unterschiedlichen Gasen, z.B. Luft, O₂, N₂, CO₂, zu begasen. Von dem Begasungssystem D aus erfolgt die Gaszuführung zu der Zellkulturkammergruppierung A mittels einer sterilen Schlauchleitung 26. Auch hierbei können die Gase von den verschiedenen Zellkulturkammern 20 unter Verwendung entsprechend zugeordneter Kanäle 4' und 4"' (vgl. Figur 3A) flexibel weitergeleitet werden.

Gaszuführung und Gasab- bzw. weiterleitung erfolgen insgesamt über sterile Schläuche, die mittels Standard-Schlauchverbinderelementen und Verteilern bei Versuchsstart installiert werden. Die Verbindungen der Schlauchverbinderelemente mit den entsprechend zugeordneten Kanälen 4' und 4"' der Membranplatte 1 sind so aufeinander abgestimmt, daß die Sterilität gewährleistet ist.

Auch bei dem Begasungssystem D können aus Flexibilitätsgründen die Gase, die Strömungsrichtungen, die Gasverteilung sowie die Begasungskonzentration während des Versuchs geändert bzw. gesteuert werden. Zu diesem Zweck ist wiederum das Begasungssystem D über eine Verbindungsleitung 39 an das computergesteuerte Überwachungs- und Steuerungssystem G angeschlossen, das die entsprechende Software für die Steuerung des Begasungssystems D enthält.

Schließlich gehört zu dem Zellkultursystem 30 noch ein Monitoring-System F, das vorgegebene Sensormodule 34 aufweist. Mit Hilfe dieses Monitoring Systems F können während der gesamten Versuchsdauer die relevanten Parameter in der jeweiligen Zellkulturkammer 20 der Zellkulturkammergruppierung A mittels entsprechend zugeordneter Sensoren gemessen, insbesondere permanent gemessen werden, wobei es sich bei diesen Parametern z.B. um pH-Wert, Glucose, Lactat, Sauerstoff, Elektropotentiale usw. handelt. Zu diesem Zweck steht das Monitoring-System F über eine Leitung 41, über einen Knotenpunkt 42 und von dort aus über weitere Leitungen 43 und 44 und entsprechend zugeordnete Abzweigleitungen mit den einzelnen Zellkulturkammern 20 der Zellkulturkammergruppierung A des Zellkultursystems 30 in Verbindung.

Die von den (nicht gezeigten) Sensoren gemessenen Parameter werden von dem Monitoring-System F über eine Leitung 35 an das computergesteuerte Überwachungs- und Steuerungssystem G zur entsprechenden Verarbeitung weitergeleitet. Wie bereits weiter oben anhand der Figuren 1 bis 3A erläutert, weist die Zellkulturkammer 20 wenigstens einen Kanal 4 für Sensorikanschluß auf, wobei die entsprechenden Sensoren und der zugeordnete Kanal 4 der Membranplatte 1 so aufeinander abgestimmt sind, daß die Sterilität gewährleistet ist.

Insgesamt betrachtet, ist das mit den erfindungsgemäßen Zellkulturkammern 20 bestückte, geschlossene Zellkultursystem 30 in der Lage, hochkomplexe biologische Vorgänge in Echtzeit und unter nahezu in-vivo-Bedingungen, d.h. wie im lebenden Organismus, zu simulieren.

## Patentansprüche

1. Zellkulturkammer (20) für ein geschlossenes Zellkultursystem zur kontinuierlichen Versorgung unterschiedlicher Zellen mit flüssigen Nährmedien, Wachstumsfaktoren, Gasen und dergleichen, **gekennzeichnet durch** die Kombination folgender Merkmale:
a) Eine Membranplatte (1) mit einer Membran (2) zur Aufnahme wenigstens einer Zellkultur und mit einer Anzahl von Kanälen (4, 4', 4", 4"') für Flüssigkeitszufuhr, Begasung und Sensorikanschluss;
b) eine auf der einen Seite der Membranplatte (1) angeordnete, lichtdurchlässige Glasscheibe (3) für eine Beobachtung des Inneren der Zellkulturkammer (20) von der genannten einen Seite aus;
c) ein System (B) zur permanenten mikroskopischen Beobachtung des Inneren der Zellkulturkammer (20) **durch** die auf der einen Seite der Membranplatte (1) angeordnete, lichtdurchlässige Glasscheibe (3) hindurch; und
d) eine auf der anderen, gegenüberliegenden Seite der Membranplatte (1) angeordnete Abschlussplatte (5) mit eingebauter, lichtdurchlässiger Glasscheibe (6) für eine Beleuchtung des Inneren der Zellkulturkammer (20) von der genannten anderen Seite aus mit Hilfe eines zugeordneten Beleuchtungssystems (24).

2. Zellkulturkammer nach Anspruch 1, **dadurch gekennzeichnet, daß** die lichtdurchlässige Glasscheibe (3) an der Membranplatte (1) für die Beobachtung des Inneren der Zellkulturkammer (20) im Bereich der Unterseite der Membranplatte (1) befestigt ist.

3. Zellkulturkammer nach Anspruch 1, **dadurch gekennzeichnet, daß** die Abschlußplatte (5) einen Zellkulturkammerdeckel mit einer fest integrierten, lichtdurchlässigen Glasscheibe (6) bildet, wobei der Zellkulturkammerdeckel an der Oberseite der Membranplatte (1) lösbar angebracht ist.

4. Zellkulturkammer nach einem der vorhergehenden Ansprüche, **dadurch gekennzeichnet, daß** sowohl der Zellkulturkammerdeckel (5) als auch die Unterseite der Membranplatte (1) eine Ausnehmung zur Aufnahme und Befestigung der entsprechenden Glasscheibe (6 bzw. 3) aufweist.

5. Zellkulturkammer nach einem der vorhergehenden Ansprüche, **dadurch gekennzeichnet, daß** die Glasscheibe (6, 3) eine Saphierglasscheibe ist.

6. Zellkulturkammer nach einem der vorhergehenden Ansprüche, **dadurch gekennzeichnet, daß** zur Fixierung der Membran (2) an der Membranplatte (1) ein Haltering (7) vorgesehen ist, der mit Hilfe des Zellkulturkammerdeckels (5) auf den Randbereich der Membran (2) preßbar ist, wodurch diese letztere fixierbar ist.

7. Zellkulturkammer nach einem der vorhergehenden Ansprüche, **dadurch gekennzeichnet, daß** auf der der Membranplatte (1) zugewendeten Seite des Zellkulturkammerdeckels (5) ein Dichtring (8) vorgesehen ist, durch den im geschlossenen Zustand der Zellkulturkammer (20) die auf der Membran (2) ausgesäte Zellkultur aseptisch verschlossen wird.

8. Zellkulturkammer nach einem der vorhergehenden Ansprüche, **dadurch gekennzeichnet, daß** durch eine geeignete Kompartimentierung der Zellkulturkammer (20) eine konstante, kontinuierliche Begasung durch die entsprechend zugeordneten Kanäle (4', 4"') hindurch mit frei wählbaren Konzentrationen unterschiedlichster Gase ermöglicht ist.

9. Zellkulturkammer nach einem der vorhergehenden Ansprüche, **dadurch gekennzeichnet, daß** die Membranplatte (1) auf ihrer dem Zellkulturkammerdeckel (5) gegenüberliegenden Seite an einer zugeordneten Halteplatte (10) zum Einbringen in das Zellkultursystem anbringbar ist, wobei diese Halteplatte (10) eine integrierte Heizung für die Zellkulturkammer (20) aufweist.

10. Zellkulturkammer nach Anspruch 9, **dadurch gekennzeichnet, daß** die Heizung eine elektrische Heizung ist.

11. Zellkulturkammer nach einem der vorhergehenden Ansprüche, **dadurch gekennzeichnet, daß** die Membran (2) eine gasdurchlässige Biofolie ist.

## Claims

1. A cell culture chamber (20) for a closed cell culture system for continuously supplying different cells with liquid nutrient media, growth factors, gases and the like, **characterized by** the combination of the following features:
a) a membrane plate (1) with a membrane (2) for holding at least one cell culture and with a number of channels (4, 4', 4", 4"') for liquid supply, gassing and sensor connection;
b) a transparent glass pane (3) arranged on one side of the membrane plate (1) for observing the interior of the cell culture chamber (20) from the aforementioned side;
c) a system (B) for permanent microscopic observation of the interior of the cell culture chamber (20) through the transparent glass pane (3) arranged on one side of the membrane plate (1); and
d) a cover plate (5) arranged on the other, opposite side of the membrane plate (1) and having an integrated, transparent glass pane (6) for illuminating the interior of the cell culture chamber (20) from the aforementioned other side by means of an associated illumination system (24).

2. The cell culture chamber according to claim 1, **characterized in that** the transparent glass pane (3) on the membrane plate (1) for observing the interior of the cell culture chamber (20) is fixed in the region of the underside of the membrane plate (1).

3. The cell culture chamber according to claim 1, **characterized in that** the cover plate (5) forms a cell culture chamber lid with a securely integrated transparent glass pane (6), wherein the cell culture chamber lid is releasably attached to the upper side of the membrane plate (1).

4. The cell culture chamber according to any one of the preceding claims, **characterized in that** both the cell culture chamber lid (5) and the underside of the membrane plate (1) has an opening for accommodating and fixing the corresponding glass pane (6 and 3).

5. The cell culture chamber according to any one of the preceding claims, **characterized in that** the glass pane (6, 3) is a sapphire glass pane.

6. The cell culture chamber according to any one of the preceding claims, **characterized in that** a retaining ring (7) is provided for fixing the membrane (2) to the membrane plate (1), which retaining ring can be pressed by means of the cell culture chamber lid (5) onto the edge region of the membrane (2), as a result of which the latter can be fixed.

7. The cell culture chamber according to any one of the preceding claims, **characterized in that** a sealing ring (8) is provided on the side of the cell culture chamber lid (5) facing towards the membrane plate (1), by means of which sealing ring the cell culture prepared on the membrane (2) is aseptically sealed in the closed state of the cell culture chamber (20).

8. The cell culture chamber according to any one of the preceding claims, **characterized in that**, by means of suitable compartmentalization of the cell culture chamber (20), it is possible to achieve a constant, continuous gassing through the respectively assigned channels (4', 4"') with freely selectable concentrations of various gases.

9. The cell culture chamber according to any one of the preceding claims, **characterized in that** the membrane plate (1), on its side opposite the cell culture chamber lid (5), can be attached to an associated retaining plate (10) for introduction into the cell culture system, wherein this retaining plate (10) has an integrated heating system for the cell culture chamber (20).

10. The cell culture chamber according to claim 9, **characterized in that** the heating system is an electrical heating system.

11. The cell culture chamber according to any one of the preceding claims, **characterized in that** the membrane (2) is a gas-permeable biofilm.

## Revendications

1. Chambre de culture cellulaire (20) pour un système de culture cellulaire fermé pour l'approvisionnement continu de différentes cellules avec des fluides nutritifs, facteurs de croissance, gaz et autres semblables, **caractérisé par** la combinaison de caractéristiques suivantes :
a) une plaque à membrane (1) avec une membrane (2) pour le logement d'au moins une culture cellulaire et avec un nombre de canaux (4, 4', 4", 4"') pour l'alimentation en liquide, le gazage et le raccordement du système de capteurs ;
b) un disque en verre (3) translucide, disposé sur un côté de la plaque à membrane (1), permettant l' observation de l'intérieur de la chambre de culture cellulaire (20) depuis le côté susmentionné ;
c) un système (B) pour l'observation microscopique permanente de l'intérieur de la chambre de culture cellulaire (20) à travers le disque en verre (3) translucide disposé sur un côté de la plaque à membrane (1) ; et
d) une plaque terminale (5), disposée de l'autre côté opposé de la plaque à membrane (1), avec disque en verre (6) translucide intégré pour l'éclairage de l'intérieur de la chambre de culture cellulaire (20) depuis l'autre côté susmentionné à l'aide d'un système d'éclairage (24) qui lui est affecté.

2. Chambre de culture cellulaire selon la revendication 1, **caractérisée en ce que** le disque en verre (3) translucide est fixé sur la plaque à membrane (1) pour l'observation de l'intérieur de la chambre de culture cellulaire (20) au niveau de la face inférieure de la plaque à membrane (1).

3. Chambre de culture cellulaire selon la revendication 1, **caractérisée en ce que** la plaque terminale (5) constitue un couvercle de chambre de culture cellulaire avec un disque en verre (6) translucide intégré fixe, étant donné que le couvercle de chambre de culture cellulaire est installé de manière amovible sur la face supérieure de la plaque à membrane (1).

4. Chambre de culture cellulaire selon l'une quelconque des revendications précédentes, **caractérisée en ce que** aussi bien le couvercle de chambre de culture cellulaire (5) que la face inférieure de la plaque à membrane (1) présentent un évidement pour le logement et la fixation du disque en verre (6 ou 3) correspondant.

5. Chambre de culture cellulaire selon l'une quelconque des revendications précédentes, **caractérisée en ce que** le disque en verre (6, 3) est un disque en verre de saphir.

6. Chambre de culture cellulaire selon l'une quelconque des revendications précédentes, **caractérisée en ce que**, pour la fixation de la membrane (2) sur la plaque à membrane (1), il est prévu une bague de maintien (7) qui peut être pressée à l'aide du couvercle de chambre de culture cellulaire (5) sur le bord de la membrane (2), ce qui permet de fixer cette dernière.

7. Chambre de culture cellulaire selon l'une quelconque des revendications précédentes, **caractérisée en ce qu'**une bague d'étanchéité (8) est prévue sur le côté du couvercle de chambre de culture (5) orienté vers la plaque à membrane (1), bague d'étanchéité au moyen de laquelle la culture cellulaire semée sur la membrane (2) est obturée de manière aseptique lorsque la chambre de culture cellulaire (20) est fermée.

8. Chambre de culture cellulaire selon l'une quelconque des revendications précédentes, **caractérisée en ce que**, par une division en compartiments adéquate de la chambre de culture cellulaire (20), un gazage constant et continu est permis à travers les canaux (4', 4"') correspondants avec des concentration pouvant être choisies librement des gaz les plus diversifiés.

9. Chambre de culture cellulaire selon l'une quelconque des revendications précédentes, **caractérisée en ce que** la plaque à membrane (1) peut être montée sur son côté opposé au couvercle de chambre de culture cellulaire (5) sur une plaque de maintien (10) qui lui est affectée en vue de l'introduction dans le système de culture cellulaire, étant donné que cette plaque de maintien (10) présente un chauffage intégré pour la chambre de culture cellulaire (20).

10. Chambre de culture cellulaire selon la revendication 9, **caractérisée en ce que** le chauffage est un chauffage électrique.

11. Chambre de culture cellulaire selon l'une quelconque des revendications précédentes, **caractérisée en ce que** la membrane (2) est une feuille biologique perméable aux gaz.
